# FASCICULE DE BREVET EUROPEEN

(11) **EP 0 901 522 B1**
(45) Date de publication et mention de la délivrance du brevet: **25.10.2006**
(21) Numéro de dépôt: 97925118.8
(22) Date de dépôt: 22.05.1997
(51) Int. Cl.: C12N 5/10

(54) **CELLULES AVIAIRES IMMORTELLES**
UNSTERBLICHE VOGELZELLEN
IMMORTAL AVIAN CELLS

(30) Priorité: 23.05.1996 FR 9606630
(43) Date de publication de la demande: 17.03.1999
(73) Titulaire: MERIAL, 69007 Lyon (FR)
(72) Inventeur: BOUQUET, Jean-François, F-69280 Sainte Consorce (FR); CLEUZIAT, Catherine, F-69003 Lyon (FR); SAMARUT, Jacques, F-69100 Villeurbanne (FR); DESMETTRE, Philippe, F-69130 Ecully (FR)
(74) Mandataire: Nargolwalla, Cyra
(86) Numéro de dépôt international: PCT/FR1997/000897
(87) Numéro de publication internationale: WO 1997/044443

(56) Documents cités:
- EP-A- 0 242 272
- WO-A-91/18971
- WO-A-92/10563
- WO-A-93/20200
- ONCOGENE, vol. 8, 1993, pages 619-624, XP002038734 GUILHOT ET AL: "THE 12S ADENOVIRAL E1A PROTEIN IMMORTALIZES AVIAN CELLS AND INTERACTS WITH THE AVIAN RB PRODUCT" cité dans la demande
- MOLECULAR AND CELLULAR BIOLOGY, vol. 7, no. 8, Août 1987, pages 2794-2802, XP002038735 NEUFELD ET AL: "IMMORTALIZATION OF HUMAN FIBROBLASTS TRANSFORMED BY ORIGIN-DEFECTIVE SIMIAN VIRUS 40" cité dans la demande
- JOURNAL OF VIROLOGY, vol. 67, no. 10, Octobre 1993, pages 6025-6032, XP002038736 NANICHE ET AL: "HUMAN MEMBRANE COFACTOR PROTEIN (CD46) ACTS AS A CELLULAR RECEPTOR FOR MEASLES VIRUS" cité dans la demande
- ONCOGENE, vol. 10, 1995, pages 1501-1509, XP002038737 ROSENBERG ET AL: "OVEREXPRESSION OF HUMAN CYCLIN A ADVANCES ENTRY INTO S PHASE" cité dans la demande
- NATURE, vol. 349, 14 Février 1991, pages 612-614, XP002038738 GREGORY ET AL: "ACTIVATION OF EPSTEIN-BARR VIRUS LATENT GENES PROTECTS HUMAN B CELLS FROM DEATH BY APOPTOSIS" cité dans la demande

## Description

La présente invention est relative à des lignées de cellules aviaires et leurs dérivés.

L'établissement de lignées cellulaires à partir d'organes prélevés sur les espèces aviaires ne peut être obtenu spontanément, comme c'est le cas avec certains organes provenant d'espèces mammifères.

Les seules lignées cellulaires disponibles jusqu'à maintenant ont été obtenues en utilisant les propriétés transformantes de certains virus aviaires ayant des propriétés oncogènes, tels que les rétrovirus du groupe leucoses aviaires ou le virus de la maladie de Marek, ou de certaines molécules chimiques telles que la méthylcholanthrène et la diéthyl-nitrosamine.

Ces lignées cellulaires présentent, pour la plupart, un caractère de transformation important qui les rend impropres à la multiplication de virus vaccinaux.

Des auteurs se sont engagés sur une nouvelle voie consistant à introduire dans les cellules un vecteur ne présentant pas de caractère oncogène mais capable d'intégrer dans ces cellules un gène choisi pour sa capacité à induire l'immortalisation.

Les premiers essais ont été réalisés à l'aide de vecteurs intégrant des gènes de rétrovirus aviaires tels que erbA, erbB.

La demande de brevet français FR-A-2 596 770 propose un procédé d'immortalisation dans lequel on infecte une culture de cellules aviaires ou de mammifères avec un vecteur ou un système ne présentant pas de caractère oncogène pour lesdites cellules mais capable d'intégrer dans ces cellules un gène choisi parmi v-myb, v-ets et v-erbA. Des vecteurs appropriés peuvent être les virus AMV, E26 et XJ12, ce dernier étant un virus dérivé du virus AEV dans lequel le gène v-erB, oncogène, a été supprimé.

Dans la pratique, ces essais ont permis d'obtenir des lignées cellulaires établies à partir de cellules de la lignée hématopoïétique, mais n'ont pas donné les résultats escomptés pour les cellules d'embryon de poulet en culture adhérente telles que les fibroblastes ou les cellules épithéliales.

Des lignées de cellules aviaires de type myéloblastoïde (cellules sanguines) non transformées ont pu être obtenues à l'aide de l'oncogène myb (demande de brevet internationale WO91/18971).

Parallèlement, des auteurs ont proposé les gènes précoces t et T du virus simien SV40 pour immortaliser des cellules provenant de différents tissus de mammifères (D.S. Neufeld et al.. Molecular and Cellular Biology, août 1987, 2794-2802, O. Kellermann et F. Kelly, Differentiation 1986, 32 : 74-81 et demande de brevet français FR-A-2 649 721).

La demande de brevet français FR-A-2 649 721 propose de son côté une méthode d'immortalisation conditionnelle qui serait utilisable pour tous les types cellulaires et dans toutes les espèces, l'objectif étant ici de remédier à l'inconvénient de la grande spécificité des voies classiques (limitation à des espèces et/ou à des types cellulaires particuliers) : transformation des cellules par un virus transformant (adénovirus, virus d'Epstein-Barr, certains papovavirus tels que le virus SV40 ou le virus du polyome ; par exemple, le virus SV40 est indiqué comme ne transformant que les cellules de rongeurs et les cellules humaines) ; transfection avec des constructions contenant un gène transformant lié à un promoteur viral ; transfection avec un gène transformant lié à un promoteur cellulaire. Le choix de cette demande de brevet se porte sur une construction associant un fragment d'ADN de la séquence régulatrice de la vimentine et un fragment d' ADN codant pour un gène immortalisant, qui peut être l'antigène T du virus SV40 sous le contrôle du promoteur, inductible, de la vimentine. Les espèces aviaires ne sont jamais mentionnées dans ce document.

Dans l'espèce aviaire, l'utilisation réelle de tels oncogènes viraux n'a jamais été décrite excepté l'utilisation de la forme 12S de la protéine E1A de l'Adénovirus 5 humain qui a permis d'immortaliser des cellules épithélioïdes de caille (Guilhot et al. (1993), Oncogene 8 : 619-624).

Contre toute attente, les inventeurs ont réussi à produire des lignées cellulaires aviaires, immortelles et non transformées.

De manière plus générale, les inventeurs ont trouvé qu'il était possible de préparer des lignées cellulaires aviaires immortelles non transformées et résistantes à l'apoptose, même à partir de cellules de tissus aviaires, c'est-à-dire autres que les cellules circulantes du sang ou hématopoïétiques.

La présente invention a donc pour objet des cellules aviaires immortalisées non transformées, résistantes à l'apoptose, en particulier provenant de tissus aviaires c'est-à-dire autres que des cellules sanguines ou hématopoïétiques, notamment fibroblastes et cellules épithéliales, par exemple d'embryons.

La présente invention a plus particulièrement pour objet une lignée cellulaire aviaire immortelle, non transformée, choisie parmi le groupe consistant en :
- lignée TDF-2A bcl-2 déposée à la CNCM (Collection Nationale de Cultures de Microoganismes de l'Institut Pasteur) sous la référence I-1709
- lignée TCF-4.10 déposée à la CNCM sous la référence I-1710
- lignée TCF-4.10 bcl-2 déposée à la CNCM sous la référence I-1711

bcl-2 signifie que les cellules de la lignée intègrent de manière fonctionnelle le gène bcl-2 qui leur confère une résistance à l'apoptose (WO-A-93/20200 incorporée ici par référence).

Bien entendu, l'invention couvre les cellules issues de ces lignées. Par cela, il faut comprendre que sont couvertes non seulement les cellules telles que déposées à la CNCM sous les références indiquées, mais aussi les cellules constituant la descendance des précédentes, d'une part celles obtenues par simple multiplication et pouvant subir des mutations lors des multiplications et d'autre part celles obtenues après modification volontaire, ce qu'on appelle alors les cellules dérivées, et bien sûr celles ayant subi les deux types de modifications.

L'invention couvre donc aussi les cellules dérivées obtenues par modifications des cellules ci-dessus. Ces modifications peuvent comprendre :
- Insertion d'une ou plusieurs cassettes d'expression comprenant chacune une ou plusieurs séquences nucléotidiques codant pour une molécule d'intérêt industriel, ces cassettes d'expression étant aptes à produire cette molécule après insertion dans les cellules de l'invention. La technique est parfaitement connue de l'homme du métier. Comme molécules d'intérêt industriel, on peut citer notamment des sous-unités virales de type peptide, protéine, glycoprotéine, notamment à usage de vaccin ou de réactif de diagnostic, des molécules protéiques telles que des hormones, etc.
- Infection chronique par un virus apte à se multiplier dans ces cellules, à des fins de production virale ou de vaccin, avec ou sans modification préalable de la sensibilité vis-à-vis de ce virus. L'infection peut aussi ne pas être chronique mais réalisée sur un lot de cellules choisi pour la multiplication virale.
   (Les modifications qui suivent s'entendent de préférence avantageusement combinées aux deux types précédents).
- Introduction de gènes de survie ou anti-apoptose autres que bcl-2 tels que les gènes codant pour les protéines p19E1B de l'adénovirus humain (Rao et al. (1992), Proc. Natl. Acad. Sci. USA 89 : 7742-7746), LMP-1 (Gregory et al. (1991), Nature 349 : 612-614) et BHRF1 (Pearson et al. (1987), Virology 160 : 151-161) du virus Epstein Barr, ICP34.5 du virus herpes simplex (Chou et Roizman (1992), Proc. Natl. Acad. Sci. USA 89 : 3266-3270) et p35 du baculovirus (Clem et al. (1991), Science 254 : 1388-1390), afin de rendre ces lignées plus résistantes aux conditions de culture, notamment maintien à confluence.
- Surexpression de gènes impliqués dans le contrôle du cycle cellulaire par des vecteurs appropriés pour augmenter la vitesse de prolifération. En effet, il a été montré que, dans certains cas, la surexpression de gènes codant pour des cyclines entraînait un raccourcissement du cycle cellulaire et donc une augmentation de la vitesse de prolifération (Rosenberg et al. (1995), Oncogene 10 : 1501-1509 ; Quelle et al. (1993), Genes and Dev. 7 : 1559-1571).
- Modification du spectre de sensibilité virale des lignées par intégration de gènes codant pour des récepteurs de virus d'intérêt, en vue de leur multiplication.
   On peut se référer à l'espèce mammifère où l'expression du récepteur du virus de la rougeole (CD46) par des cellules murines, normalement non permissives au virus, entraîne une sensibilité de ces cellules à ce virus et la capacité à le répliquer (Naniche et al. (1993), J. Virol. 67 : 6025-6032). L'intérêt est notamment de rendre les cellules sensibles à un virus afin de le produire sur celles-ci.
- Intégration d'oncogènes aptes à accélérer la croissance cellulaire.

Il va de soi que les cellules dérivées selon l'invention peuvent comprendre une ou plusieurs des modifications présentées ci-dessus.

L'invention a encore pour objet un procédé de production de molécules d'intérêt industriel ou de virus, comprenant la culture des cellules décrites ci-dessus.

Dans le cadre de la présente invention, on s'oriente notamment vers la production de molécules ou de virus pour la réalisation de réactifs de diagnostic ou de vaccins, ou encore de molécules d'intérêt thérapeutique.

L'invention va être maintenant décrite plus en détail à l'aide de modes de réalisation pris à titre d'exemples non limitatifs et se référant au dessin annexé dans lequel :
- la figure 1 montre la structure du vecteur pDAMT servant à préparer la lignée TDF-2A, avec :
   LTR : séquence répétée directe (Long Terminal Repeat)
   ∂ LTR : LTR délétée
   MTI : promoteur Metallothionéine I murin
   SV40 T+t : région précoce SV40
   SV40 : promoteur SV40
- la figure 2 montre la structure du vecteur pphMT servant à préparer la lignée TC F-4.10, avec :
   LTR : séquence répétée directe (Long Terminal Repeat)
   phléo : gène de résistance à la phléomycine
   SV40pA : polyASV40
   MTI : promoteur Metallothionéine I murin
   SV40 T+t : région précoce SV40

### EXEMPLE 1 = Production de la lignée-cellulaire TDF-2A

### I. Description de son origine et de ses caractéristiques

### 1.1 Description du vecteur utilisé : vecteur pDAMT

Il comporte la région précoce du virus SV40 (code pour les antigènes T et t) (fragment HindIII/BamHI) (Fiers et al.(1978), Nature 273 : 113-120) sous le contrôle du promoteur métallothionéine I de souris (fragment EcoRI/BglII transformé en site HindIII) (Durnam et al.(1980), Proc. natl. Acad. Sci. USA 77 : 6511-6515 ; Brinster et al. (1982), Nature 296 : 39-42).

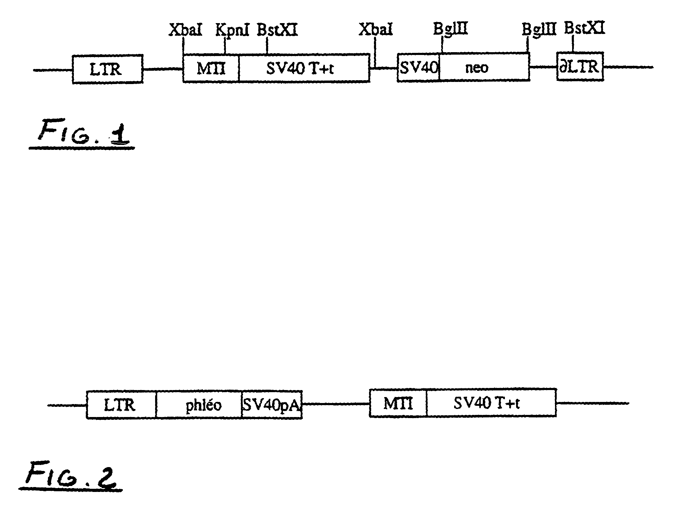

Le fragment EcoRI/EcoRI contenant cette unité transcriptionnelle provenant du vecteur pMTSVneo (Peden et al. (1989), Exp. Cell. Res. 185 : 60-72) a été inséré dans le site Xbal du vecteur pDA1 (Aubert et al. (1991), J. Cell. Biol. 113 : 497-506). Ce dernier dérive essentiellement du génome du virus associé au sarcome de Rous-2 (RAV-2) après modification de la LTR située en 3'. En effet, la région U3 de la LTR 3' de RAV-2 a été délétée et liée aux régions R et U5 isolées de la LTR du virus associé au sarcome de Rous-1 (RAV-1). II porte également une unité transcriptionnelle contenant le gène de résistance à la néomycine sous le contrôle du promoteur SV40 dérivant du vecteur pSV2neo (Southern et Berg (1982), J. Mol. Appl. Genet. 1 : 327-341). Voir figure 1.

### 1.2. Etablissement de la lignée et démonstration du caractère immortalisé.

Des cellules provenant d'embryons de canard de Barbarie de 14 jours ont été transfectés par le vecteur pDAMT par la méthode utilisant le diméthylsulfoxide (DMSO) et décrite par Kawai et Nishizawa (1984), Mol. Cell. Biol. 4 : 1172-1174. Les cellules transfectées sont ensuite sélectionnées par application de généticine G418 (150 µg/ml) pendant 15 jours. Les clones résistants sont alors subcultivés régulièrement à raison de 1 à 2 passages par semaine. Après cette période de prolifération active de 3 mois, les cellules sont entrées dans une période de crise durant laquelle la plupart des cellules sont mortes. Après cette période qui a duré environ 2 mois, plusieurs clones ont repris une prolifération active suggérant leur immortalisation.

La lignée cellulaire TDF-2A est issue ainsi de 2 cultures.
Elle a été étudiée de manière plus approfondie.

Les cellules TDF-2A ont atteint 200 passages soit environ 460 générations et ont été ainsi maintenues en culture pendant plus de 600 jours en continu. Comparativement, des cellules témoins, n'exprimant pas la région précoce du virus SV40 ne peuvent être maintenus en culture plus de 20 passages.

### 1.3. Caractéristiques de prolifération.

Les cellules immortalisées sont cultivées à 38°C, en flacon roulant, dans un milieu contenant du HAM F-10 10X à 6%, 199 HANKS 10X à 4%, Tryptose Broth Phosphate 2.95 % à 4%, Bicarbonate de sodium 5,6% à 2,5%, vitamine BME 100X à 0,1%, sérum de veau foetal à 3%, Kanamycine 5% à 1%, Vancomycine 0,5% à 1%.

Dans ces conditions, leur taux de doublement est de 1 par 24 heures.

### 1.4. Expression de l'antigène T.

Par immunofluorescence ou immunophosphatase indirectes en utilisant un anticorps spécifique de l'antigène T (Pab 101 : Santa Cruz Biotechnology ref. sc147), il a été vérifié que toutes les cellules expriment l'antigène T dans leur noyau, indiquant qu'elles ont toutes intégré le vecteur.

Cette intégration a d'ailleurs été montrée par Southern-blot. L'ADN génomique des fibroblastes immortalisés a été digéré par les enzymes de restriction Xbal, BstXI. L'hybridation avec une sonde spécifique de l'antigène T (fragment NdeI/NdeI de 1018 pb) a permis de vérifier que l'unité transcriptionnelle permettant l'expression du gène immortalisant, insérée dans les cellules TDF-2A, n'avait pas subi de remaniements majeurs. En effet, la taille des fragments d'hybridation obtenus est conforme à celle attendue.

### 1.5. Absence de pouvoir tumorigène.

Les cellules immortalisées ne présentent pas de pouvoir tumorigène. Elles sont incapables de former des colonies en milieu semi-solide ou de former des tumeurs sur membrane chorioallantoïdienne d'oeufs de poule ou de cane. Elles sont également incapables de former des tumeurs sur souris nue (souris "nude"), sur poussins et canetons SPF (exempts d'organismes pathogènes) de 1 jours.

### 1.6. Caryotype.

Le caryotype des cellules TDF-2A a été étudié aux 114ème et 135ème passages. Il a permis de vérifier que les cellules étaient bien d'origine aviaire avec la présence de microchromosomes caractéristiques de cette espèce. De plus, les chromosomes observés sont représentatifs des chromosomes rencontrés dans les cellules primaires d'embryons de canard confirmant ainsi l'origine de la lignée.

### II. Propriétés.

Les cellules TDF-2A présentent notamment une sensibilité aux virus spécifiques du canard tels que l'adénovirus, le parvovirus et le reovirus qui sont habituellement répliqués sur cellules primaires d'embryons de canard. On peut donc produire ces virus sur cette lignée.

### EXEMPLE 2 : Caractérisation de la lignée TDF-2A par identification des sites d'intégration.

L'ADN génomique des cellules TDF-2A, préparé à partir des cellules provenant du 114ième et du 135ième passages, a été digéré par les enzymes de restriction BgIII et KpnI. L'ADN ainsi traité est alors soumis à une électrophorèse sur gel, suivie d'un transfert sur membrane de nylon, puis hybridé avec une sonde spécifique de l'antigène T (fragment Ndel/Ndel de 1018 pb). Ainsi, la digestion par BgIII permet d'obtenir deux bandes d'hybridation de haute taille (d'environ 15 et 23 kb) suggérant l'existence de deux sites d'intégration. La digestion par Kpnl entraîne l'obtention d'une bande majoritaire de haute taille (environ 20 kb) et d'au moins une bande minoritaire confirmant l'existence d'au moins deux sites d'intégration.

### EXEMPLE 3 : Production de la lignée cellulaire TCF-4.10

### 1. Description de son origine et de ses caractéristiques

### 1.1. Description du vecteur utilisé : vecteur pphMT

Il comporte la région précoce du virus SV40 (code pour les antigènes T et t) (fragment HindIII/BamHI) (Fiers et al. (1978), Nature 273 : 113-120) sous le contrôle du promoteur métallothionéine I de souris (fragment EcoRI/BgIII transformé en site HindIII) (Durnam et al. (1980), Proc. natl. Acad. Sci. USA 77: 6511-6515 ; Brinster et al. (1982), Nature 296 : 39-42).

Le fragment EcoRI/EcoRI contenant cette unité transcriptionelle provenant du vecteur pMTSVneo (Peden et al. (1989), Exp. Cell. Res. 185 : 60-72) a été inséré dans le site EcoRI du vecteur pUT507 (commercialisé par CAYLA-FRANCE) situé en 3' de la région permettant l'expression du gène de résistance à la phléomycine (figure 2). La structure du vecteur pUT507 est décrite dans Mulsant et al. (1988), Somatic Cell and Molecular genetics 14 : 243-252.

### 1.2. Etablissement de la lignée et démonstration du caractère immortalisé.

Des fibroblastes provenant d'embryons de poulet ont été transfectés par le vecteur pphMT par la méthode utilisant le diméthylsulfoxide (DMSO) et décrite par Kawai et Nishizawa (1984), Mol. Cell. Biol. 4 : 1172-1174. Les cellules transfectées sont ensuite sélectionnées par application progressive (de 10 µg/ml à 50 µg/ml) de phléomycine pendant 15 jours. Les clones résistants sont alors subcultivés régulièrement à raison de 1 à 2 passages par semaine. Après une période de prolifération active d'environ 2 mois, les cellules sont entrées dans une période de crise où la croissance cellulaire est très faible et durant laquelle la mortalité est très élevée. Après une période qui a durée de 3 à 4 mois, quelques cellules du clone TCF-4.10 ont repris une prolifération active suggérant leur immortalisation.

Les cellules TCF-4.10 ont atteint ainsi 200 passages en culture soit environ 400 générations et ont été maintenues en culture pendant 3 années. Comparativement, des fibroblastes témoins, n'exprimant pas la région précoce du virus SV40 ne peuvent être maintenus en culture plus de 20 à 30 passages.

### 1.3. Caractéristiques de prolifération.

Les fibroblastes immortalisés sont cultivés à 38°C dans un milieu HAM F-10 10X à 6%, 199 HANKS 10X à 4%, Tryptose Broth Phosphate 2,95% à 4%. Bicarbonate de sodium 5,6% à 2,5%, vitamine BME 100X à 0,1 %, sérum de veau foetal à 3%, Kanamycine 5% à 1%, Vancomycine 0,5% à 1%. Dans ces conditions, leur taux de doublement est de 0,7 par 24 heures.

### 2.2. Expression de l'antigène T.

Par immunofluorescence ou immunophosphatase indirectes en utilisant un anticorps spécifique de l'antigène T (Pab 101 : Santa Cruz Biotechnology ref. sc147), il a été vérifié que toutes les cellules expriment l'antigène T dans leur noyau, indiquant qu'elles ont toutes intégrés le vecteur.

### 2.3. Absence de pouvoir tumorigène.

Les fibroblastes immortalisés ne présentent pas de pouvoir tumorigène. Ils sont incapables de former des tumeurs sur membrane chorioallantoïdienne d'oeufs de poule ou de cane.

### 3. Propriétés.

Les cellules TCF-4.10 présentent notamment une sensibilité aux virus aviaires. On peut citer notamment les Poxvirus aviaires tels que le Canary-pox, le Fowl-Pox, ou encore les virus de la maladie de Marek (sérotypes 1, 2 et 3(HVT)), le virus de la maladie de Gumboro. On peut donc produire ces virus sur cette lignée.

### EXEMPLE 4 : Multiplication du Canary-Pox sur cellules TCF-4.10.

Les cellules TCF-4.10 sont ensemencées en flacon roulant. Le Canary-Pox est inoculé sur tapis établi. Lorsque l'effet cytopathique engendré par le virus est généralisé, la récolte est effectuée par agitation de façon à décoller le tapis cellulaire. Celle-ci est donc composée du tapis cellulaire et de surnageant de culture. L'ensemble est homogénéisé par un traitement ultraturrax pendant 1 mn à 13 500 tours/mn (appareil IKA type T25).

La détermination du titre viral infectieux est réalisée en microméthode sur plaque de 96 puits. Les dilutions de virus sont inoculées sur tapis établi de cellules secondaires d'embryons de poulet. Chaque dilution virale est inoculée sur 6 cupules. Les plaques sont placées en incubation dans un incubateur à CO₂ pendant 8 jours. La présence de virus dans les cupules est contrôlée au microscope en observant l'effet cytopathique (ECP) caractéristique. Le titre infectieux est calculé selon la méthode de KARBER et est exprimé par le logarithme de l'inverse de la dilution virale donnant 50% d'ECP [Titre = d+r/Nx(n+N/2)] avec d égal à la dilution exprimée en log où il y a 100% de cupules positives, r égal à la raison de la dilution, N égal au nombre de cupules par dilution et n égal au nombre de cupules positives entre 0 et 100%).
Résultats : Les titres viraux obtenus sont équivalents à ceux obtenus sur cellules primaires d'embryons de canard.

### EXEMPLE 5 : Intégration du gène bcl-2

Un vecteur permettant l'expression du gène bcl-2 sous le contrôle du promoteur CMV (Cytomegalovirus humain) est transfecté dans les cellules TDF-2A et TCF-4.10 en utilisant les méthodes classiques de transfection (méthode au DMSO décrite par Kawai et Nishizawa (1984), Mol. Cell. Biol. 4 : 1172-1174 ou lipofectamine suivant les recommandations du fournisseur GIBCO-BRL).

Après sélection des cellules transfectées, l'expression de la protéine Bcl-2 est détectée par western-blot.

Les cellules expnmant la protéine Bcl-2 sont alors testées pour leur capacité à survivre dans des conditions de culture où un processus d'apoptose est observé (maintien des cellules à confluence).

Ainsi, dans le cas des cellules TDF-2A bcl-2. le processus d'apoptose engendré par l'arrivée des cellules à confluence est différé de 3 à 4 jours par rapport aux cellules TDF-2A. Dans le cas des cellules TCF-4.10 bcl-2, une augmentation de la densité cellulaire à confluence est observée par rapport aux cellules TCF-4.10.

## Revendications

1. Une cellule aviaire immortalisée, non transformée, qui intègre dans son génome la région précoce du virus SV40 codant pour les antigènes T et t et contient un gène codant pour une protéine anti-apoptose, la région précoce du virus SV40 codant pour les antigènes T et t et le gène codant pour la protéine anti-apoptose ayant été introduits dans la cellule par transfection.

2. La cellule selon la revendication 1 dans laquelle la protéine anti-apoptose est bcl-2.

3. La cellule de la revendication 1 dans laquelle la protéine anti-apoptose est p19EIB de l'adénovirus humain, LMP-1 du virus Epstein Barr, BHRF1 du virus Epstein Barr, ICP34.5 du virus herpes simplex ou p35 du baculovirus.

4. La cellule de l'une quelconque des revendications 1 à 3 dans laquelle la région précoce du virus SV40 codant pour les antigènes T et t est sous le contrôle du promoteur MTI.

5. La cellule selon l'une quelconque des revendications 1 à 4 obtenue à partir de tissus aviaires.

6. La cellule de la revendication 5 obtenue à partir de fibroblastes ou de cellules épithéliales.

7. Une lignée cellulaire comprenant des cellules selon l'une quelconque des revendications 1 à 6.

8. Une lignée cellulaire aviaire immortelle, non transformée, qui est choisie dans le groupe consistant en :
- lignée TDF-2A bcl-2 déposée à la CNCM (Collection Nationale de Cultures de Microorganismes de l'Institut Pasteur) sous la référence I-1709,
- lignée TCF-4.10 déposée à la CNCM sous la référence I-1710,
- lignée TCF-4.10 bcl-2 déposée à la CNCM sous la référence I-1711.

9. Une cellule aviaire immortelle, non transformée, dérivée d'une lignée cellulaire selon la revendication 8.

10. La cellule selon l'une quelconque des revendications 1 à 6 et 9, comprenant l'intégration d'un gène codant pour un récepteur viral.

11. La cellule selon l'une quelconque des revendications 1 à 6 et 9, comprenant l'intégration d'oncogène apte à accélérer la croissance cellulaire.

12. La cellule selon l'une quelconque des revendications 1 à 6 et 9 à 11, contenant en outre une séquence nucléotidique hétérologue.

13. La cellule selon la revendication 12 dans laquelle la cellule exprime un produit codé par la séquence nucléotidique hétérologue.

14. La cellule de la revendication 13 dans laquelle la séquence nucléotidique code pour un peptide, une protéine ou une glycoprotéine virale.

15. La cellule de la revendication 14 dans laquelle la séquence nucléotidique code pour une hormone.

16. La cellule selon l'une quelconque des revendications 1 à 6 et 9 à 11, infectée par un virus.

17. Cellule de la revendication 16 établie à partir d'une cellule provenant d'embryon de canard.

18. La cellule de la revendication 17 dans laquelle le virus est un virus du canard sélectionné notamment parmi l'adénovirus de canard, le parvovirus du canard et un réovirus.

19. Cellule de la revendication 16 établie à partir d'un fibroblaste provenant d'embryon de poulet.

20. Cellule de la revendication 19 dans laquelle le virus est un virus aviaire sélectionné notamment parmi les poxvirus, tels que le Canary-pox, le Fowl-pox, les virus de la maladie de Marek (sérotype 1, 2), le virus herpès de la dinde (HVT) et un virus de la maladie de Gumboro.

21. Une méthode pour produire un virus, comprenant l'infection de la cellule selon l'une quelconque des revendications 1 à 6 et 9 à 11 avec le virus, dans des conditions permettant la production du virus.

22. Une méthode pour produire un peptide, protéine ou une glycoprotéine virale, comprenant l'infection de la cellule selon l'une quelconque des revendications 1 à 6 et 9 à 11 avec le virus, dans des conditions permettant la production du peptide, de la protéine ou de la glycoprotéine virale.

23. La méthode de la revendication 22, dans laquelle la cellule est une cellule selon l'une des revendications 18 ou 20.

## Claims

1. An untransformed, immortalized, avian cell that integrates into its genome the early region of SV40 encoding the T+t antigens, and contains a gene encoding an anti-apoptotic protein, the early region of SV40 encoding the T+t antigens and the gene encoding an anti-apoptotic protein are introduced into the cell by transfection.

2. The cell of any one of claim 1 wherein the anti-apoptotic protein is bcl-2.

3. The cell of any one of claim 1 wherein the anti-apoptotic protein is selected from the group consisting of human adenovirus p19E1B, Epstein Barr virus LMP-1, Epstein Barr virus BHRF1, simplex herpesvirus ICP34.5, and baculovirus p35.

4. The cell of claim 4 wherein the early region of SV40 encoding the T+t antigens is under the control of the MTI promoter.

5. The cell of any one of the claims 1 to 4 obtained from avian tissue.

6. The cell of claim 5 obtained from fibroblasts or epithelial cells.

7. A cell line comprising cells of any one of claims 1 to 6.

8. Immortal, untransformed avian cell line, which is selected from the group consisting of :
- the TDF-2A bcl-2 cell line, which is deposited in the CNCM (Pasteur Institute National Collection of Microorganism Cultures) under the reference number I-1709,
- the TCF-4.10 cell line, which is deposited in the CNCM (Pasteur Institute National Collection of Microorganism Cultures) under the reference number I-1710,
- the TCF-4.10 bc12 cell line, which is deposited in the CNCM (Pasteur Institute National Collection of Microorganism Cultures) under the reference number I-1711.

9. An untransformed, immortalized avian cell which is derived from a cell line according to claim 8.

10. The cell of any one of claims 1 to 6 and 9, comprising the integration of a gene encoding a viral receptor.

11. The cell of any one of claims 1 to 6 and 9, comprising the integration of oncogene able to accelerate cell growth.

12. The cell of any one of claims 1 to 7 and 9 to 11, further containing a heterologous nucleotide sequence.

13. The cell of claim 12 which express a product encoded by the heterologous nucleotide sequence.

14. The cell of claim 13 wherein the nucleotide sequence encodes a viral peptide, protein, or glycoprotein.

15. The cell of claim 14 wherein the nucleotide sequence encodes a hormone.

16. The cell of any one of claims 1 to 6 and 9 to 11 infected with a virus.

17. The cell of claim 16 generated from a duck embryo-derived cell.

18. The cell of claim 17 infected with a duck virus, in particular a duck adenovirus, a parvovirus and a reovirus.

19. The cell of claim 16 generated from a chicken embryo-derived fibroblasts.

20. The cell of claim 19 infected with an avian virus selected from the group consisting of poxvirus such as the canarypox, fowlpox, the Marek's Disease Virus serotype 1 and 2, herpes virus of turkey (HVT) and Gumboro disease virus.

21. A method for producing a virus comprising infecting the cell of any one of claims 1 to 6 and 9 to 11 with the virus under conditions allowing the production of the virus.

22. A method for producing a viral peptide, protein or glycoprotein comprising infecting the cell of any one of claims 1 to 6 and 9 to 11 with the virus, under conditions allowing the production of the viral peptide, protein or glycoprotein.

23. The method of claim 22 in which the cell is a cell of any one of claims 18 or 20.

## Patentansprüche

1. Immortalisierte, nicht transformierte Vogelzelle, die in ihrem Genom die frühe Region des SV40-Virus, die die Antigene T und t codiert, integriert enthält und die ein Gen enthält, das ein Anti-Apoptose-Protein codiert, wobei die frühe Region des SV40-Virus, die die Antigene T und t codiert, und das Gen, das das Anti-Apoptose-Protein codiert, durch Transfektion in die Zelle eingeführt wurden.

2. Zelle gemäß Anspruch 1, wobei das Anti-Apoptose-Protein blc-2 ist.

3. Zelle gemäß Anspruch 1, wobei das Anti-Apoptose-Protein p19E1 B des humanen Adenovirus, LMP-1 des Epstein-Barr-Virus, BHRF1 des Epstein-Barr-Virus, ICP34.5 des Herpes-simplex-Virus oder p35 des Baculovirus ist.

4. Zelle gemäß einem der Ansprüche 1 bis 3, wobei die frühe Region des SV40-Virus, die die Antigene T und t codiert, durch den MTI-Promotor kontrolliert wird.

5. Zelle gemäß einem der Ansprüche 1 bis 4, die ausgehend von Geflügelgewebe erhalten wurde.

6. Zelle gemäß Anspruch 5, die ausgehend von Fibroblasten oder Epithelialzellen erhalten wurde.

7. Zelllinie, die Zellen gemäß einem der Ansprüche 1 bis 6 umfasst.

8. Zelllinie immortalisierter, nicht transformierter Vogelzellen ausgewählt aus der Gruppe bestehend aus:
- Zelllinie TDF-2A bcl-2 hinterlegt bei der CNCM (Collection Nationale de Cultures de Microorganismes de l'Institut Pasteur) unter der Hinterlegungsnummer I-1709
- Zelllinie TCF-4.10 hinterlegt bei der CNCM unter der Hinterlegungsnummer I-1710
- Zelllinie TCF-4.10 bcl-2 hinterlegt bei der CNCM unter der Hinterlegungsnummer I-1711

9. Immortalisierte, nicht transformierte Vogelzelle, die von einer Zelllinie gemäß Anspruch 8 abgeleitet ist.

10. Zelle gemäß einem der Ansprüche 1 bis 6 und 9, umfassend die Integration eines Gens, das einen viralen Rezeptor codiert.

11. Zelle gemäß einem der Ansprüche 1 bis 6 und 9, umfassend die Integration eines Oncogens, das geeignet ist, das Zellwachstum zu beschleunigen.

12. Zelle gemäß einem der Ansprüche 1 bis 6 und 9 bis 11, die zusätzlich eine heterologe Nucleotidsequenz enthält.

13. Zelle gemäß Anspruch 12, wobei die Zelle ein von der heterologen Nucleotidsequenz codiertes Produkt exprimiert.

14. Zelle gemäß Anspruch 13, wobei die Nucleotidsequenz ein Peptid, ein Protein oder ein virales Glycoprotein codiert.

15. Zelle gemäß Anspruch 14, wobei die Nucleotidsequenz ein Hormon codiert.

16. Zelle gemäß einem der Ansprüche 1 bis 6 und 9 bis 11, die mit einem Virus infiziert ist.

17. Zelle gemäß Anspruch 16, die ausgehend von einer Zelle erhalten wurde, die von einem Entenembryo abstammt.

18. Zelle gemäß Anspruch 17, wobei das Virus ein Virus der Ente ist, das insbesondere ausgewählt ist aus dem Adenovirus der Ente, dem Parvovirus der Ente und einem Reovirus.

19. Zelle gemäß Anspruch 16, die ausgehend von einem Fibroblasten erhalten wurde, der von einem Hühnerembryo abstammt.

20. Zelle gemäß Anspruch 19, wobei das Virus ein Geflügelvirus ist, das insbesondere ausgewählt ist aus der Gruppe der Pockenviren, wie z.B. Kanarienpockenvirus, Geflügelpockenvirus, Viren der Marek'schen Krankheit (Serotyp 1, 2), Puten-Herpes-Virus (HVT) und einem Virus der Gumboro-Krankheit.

21. Verfahren zur Herstellung eines Virus, umfassend die Infizierung der Zelle gemäß einem der Ansprüche 1 bis 6 und 9 bis 11 mit dem Virus unter Bedingungen, die die Herstellung des Virus erlauben.

22. Verfahren zur Herstellung eines Peptids, eines Proteins oder eines viralen Glycoproteins, umfassend die Infizierung der Zelle gemäß einem der Ansprüche 1 bis 6 und 9 bis 11 mit dem Virus unter Bedingungen, die die Herstellung des Peptids, des Proteins oder des viralen Glycoproteins erlauben.

23. Verfahren gemäß Anspruch 22, wobei die Zelle eine Zelle gemäß einem der Ansprüche 18 oder 20 ist.
